# EUROPEAN PATENT APPLICATION

(11) **EP 3 168 611 A1**
(43) Date of publication of application: **17.05.2017**
(21) Application number: 15306794.7
(22) Date of filing: 12.11.2015
(51) Int. Cl.: G01N 27/48, G01N 17/02, G01N 33/18

(54) **CORROSION OR SCALE INHIBITOR DOSAGE IN INDUSTRIAL WATER**

(71) Applicant: Total SA, 92400 Courbevoie (FR)
(72) Inventor: Moulie, Grégory, 64000 Pau (FR); Gleyzes, Christine, 64410 Billere (FR); Cugnet, Cyril, 64000 PAU (FR)
(74) Representative: Lavoix

(57) **Abstract**

The present invention relates to a method for determining a concentration of a corrosion or scale inhibitor in a water sample comprising at least one corrosion or scale inhibitor, wherein said method comprises measuring the current as a function of the potential of one or more water samples thereby determining the concentration of said corrosion or scale inhibitor in said water sample.

The present invention also relates to a method for determining the potential of a water sample comprising a corrosion or scale inhibitor, and a method for controlling or monitoring the concentration of one or more corrosion or scale inhibitors in an industrial fluid, and a kit for voltammetry analysis.

## Description

The present invention relates to a method for determining a concentration of corrosion or scales inhibitors in a water sample. Especially, the method of the present invention is applicable to the dosage of such inhibitors in connection with the oil and gas industry.

### STATE OF THE ART

Typically, the above-mentioned industry use corrosion scale inhibitors to inhibit corrosion, or deposition of various species, for example such as scale, in equipment involved in gas or petroleum extraction from soil or ground. More generally, industrial water may also contain corrosion or scale inhibitors in order to protect the equipment from corrosion and/or deposition of various species, such as scale.

One important problem in the industry is the difficulty to determine the amount of such inhibitors in water, especially industrial water. For operational and environmental reasons, it is important to quantify the amount of such inhibitors in industrial water, which may be released in the environment. Nowadays the control of the treatment by corrosion inhibitors is performed by colorimetric methods which present a low reliability and is not satisfactory. Typically, the prior art uses the methyl orange method which involves chemical extraction and spectrometry. Such a method is also limited because of the high degree of uncertainty of measurement of the amount of said inhibitors and because of its low compatibility with certain types of inhibitors. The prior art discloses analytic methods which are difficult to implement, notably because of the use of spectrometry such as mass spectrometry usually coupled to chromatography techniques. If such method can be satisfactorily used in labs equipped with such heavy facilities, they require long time (at least to set up) and are not applicable on site, especially on an industrial site, especially a platform, for example oil or gas platforms.

The technical problem of uncertainty of the amount of inhibitors in industrial water becomes an environmental problem if production water is released in the environment, typically in natural waters such as in the sea, river, lake or ocean. Corrosion or scale inhibitors are typically organic products that would pollute the environment. Therefore, it is desirable to minimize such inhibitor release in the environment and thereby important to precisely determine the amount of such inhibitors prior to industrial water release.

Also for protecting the equipment from corrosion or scale which would degrade the equipment, it is necessary to determine and monitor precisely the amount of inhibitors in industrial water in contact with such equipment.

According to the current practice to titrate corrosion inhibitor, methyl orange method uses chloroform which also has a negative impact on human body. It is desirable to avoid the use of such compounds.

The exploitation of oil or gas fields or oil recovery can be improved by injecting water into the reservoir via an injection well so as to push the oil outside the basement by at least one other well called production well. The injected water can be produced water, i.e. corrosive water due to dissolved CO₂ or H₂S may cause corrosion on facilities. Scale formation is also likely depending on water composition and physical parameters. It is thereby necessary to inhibit such these fluids by using corrosion or scale. The titration of these inhibitors, is an essential aspect of the petroleum or hydrocarbon production, to ensure they are present in sufficient amount to fulfil their function and, if necessary, to inject in good time an additional amount of such inhibitors, adjusted for the economic constraints of the process and its environmental impact. Also, the development of rapid and reliable detection methods runs further to the fact that these inhibitors are usually so efficient that they are present in the water only up to a few ppm and the presence in the operating water of very different compounds such as salts and organic residues.

In order to solve some of the above technical problems, TOTAL SA company has already developed a method based on the marking of a corrosion or scale inhibitor with a lanthanide and its detection by fluorospectrometry (WO 2015092310).

Such method involves spectrometry equipment which is not easy to have on an industrial site.

### AIMS OF THE INVENTION

The present invention aims to solve one or more of, preferably all, above-recited technical problems.

In particular, the invention aims to provide an easy way to determine the amount of corrosion or scale inhibitors in water, especially industrial water.

The present invention also aims to provide a method which avoids heavy analysis, such as mass spectrometry, difficult to implement on an industrial site, especially on an oil or gas platform.

The present invention aims to provide a method which would be easily used on site, especially on an oil and/or gas production plant.

The present invention aims to provide the method for selecting and qualifying corrosion or scale inhibitors.

The present invention aims to provide a method which is reliable for determining the amount of corrosion or scale inhibitor in water.

The invention aims to provide a method which would be independent from, or at least less dependent to, the nature of the corrosion or scale inhibitor.

The present invention aims to provide a method which is more environmentally friendly.

The present invention also aims to provide a method which would avoid the use of chloroform.

### SUMMARY OF THE INVENTION

The inventors have discovered that one or more of, preferably all, above technical problems may be overcome by implementing a method for determining a concentration of a corrosion or scale inhibitor in a water sample comprising water and at least one corrosion or scale inhibitor, said method comprising measuring the current as a function of the potential of one or more water samples thereby determining the concentration of said corrosion or scale inhibitor in said water sample.

The present invention also relates to a method for determining the potential of a water sample, wherein said method comprises preparing at least one electrode comprising a solid surface comprising mercury, putting said electrode in contact with said water sample, optionally diluted, and measuring the current as a function of the potential of said water sample, optionally diluted, thereby determining the potential of said water sample.

The present invention also relates to a method for controlling or monitoring the concentration of corrosion or scale inhibitors in produced water, comprising implementing a method as previously described.

Also the present invention relates to the preparation of an electrode especially for use in the above described methods for dosage of corrosion or scale inhibitors, and relates also to the electrode obtained.

The present invention relates to the use of a screen printed carbon electrode having a solid mercury (Hg) layer surface for measuring the current as a function of the potential of one or more water samples, optionally diluted and/or concentrated, containing at least one corrosion or scale inhibitor.

The present invention also relates to a method for producing gas or petroleum comprising implementing a method as described above in the present invention.

The present invention also relates to a kit for voltammetry analysis, said kit comprising (i) an electrolyte solution, preferably comprising KNO₃, (ii) a pH buffer solution, preferably comprising an acetate buffer solution, (iii) a carbon electrode comprising a solid layer of mercury or a carbon electrode and a solution of Hg²⁺, for example comprising HNO₃, optionally (iv) one or more standard solutions, preferably comprising a determined corrosion or scale inhibitor at a known or predetermined concentration or amount, and optionally (v) a cleaning solution, preferably comprising HNO₃.

### DETAILED DESCRIPTION OF THE INVENTION

The present invention relates in particular to a method comprising:
(a) collecting one or more water samples 10 containing water and at least one corrosion or scale inhibitor 40;
(b) optionally diluting one or more water samples 10 to prepare one or more water diluted samples 20, thereby providing one or more water samples 10, optionally diluted 20;
(c) preparing concentrated samples 30 presenting a higher corrosion or scale inhibitor concentration than said water samples 10, optionally diluted 20;
(d) measuring the current as a function of the potential of one or more water samples 10, optionally diluted 20, and of said one or more concentrated samples 30;
(e) thereby determining the concentration of said corrosion or scale inhibitor 30 in said one or more water samples 10.

The step (a) of collecting one or more water samples 10 is performed according to the techniques known in the art;

Voltammetry is a category of electroanalytical methods used in analytical chemistry and various industrial processes. Voltammetry is known and there is no difficulty for a skilled person to work such a method. Voltammetry was nevertheless not used for the analysis of corrosion or scale inhibitors according to inventor's knowledge. It was not obvious for a skilled person that voltammetry could be used for determining the concentration of corrosion or scale inhibitors concentration. Typically, such voltammetry methods involve a three electrode system, including a working electrode, an auxiliary electrode and a reference electrode. The purpose of voltammetry is to get the current as a function of potential (these curves are noted I = f(E) and typically called voltammograms). In voltammetry, the potentially varied either step by step or continuously depending on the type of method applied. Polarography is a specific type of voltammetry wherein the working electrode is a dropping mercury electrode (DME).

Accordingly, the method of the present invention and especially step (d) of measuring the current as a function of the potential of one or more water samples, optionally diluted and of said one or more concentrated samples is preferably performed by voltammetry, and preferably by preferably by polarography, cyclic voltammetry (CV), differential pulse voltammetry (DPV), or squarewave voltammetry (SWV).

In one embodiment such a measure is performed by polarography (DME).

In particular, such a measure is performed by cyclic voltammetry (CV), differential pulse voltammetry (DPV), or squarewave voltammetry (SWV).

According to one preferred embodiment, the method of the invention is performed using pulsed current.

According to a particular embodiment, step (d) is specifically performed by DPV.

According to a particular embodiment, step (d) is specifically performed by SWV.

CV, PDV, SWV are preferred over polarography because polarography involves the use of mercury in the form of liquid metallic species. This technique is very basic in the lab but the use of liquid mercury (Hg) has preferably to be avoided in particular on an industrial site, in particular on a gas or petroleum production site. Moreover, it has been surprisingly discovered that CV, PDV, SWV are preferred over DME because no, or much less interference occur during the analysis of water containing one or more corrosion or scale inhibitors.

The present invention also aims to avoid using liquid mercury (Hg) such as used in polarography.

According to one embodiment, said measure of the current as a function of the potential at step (d) is performed in the presence of at least one electrode comprising a solid surface comprising mercury.

According to one embodiment, said electrode comprising a solid surface comprising mercury is prepared *in situ,* by mercury electrodeposition on the surface of an electrode, preferably a carbon electrode.

Advantageaously, step (b) comprises adding a buffer solution to said water samples 10, optionally diluted 20.

Steps (b) and/or (d) may be implemented in the presence of a pH buffer at an appropriate pH for the corrosion or scale inhibitors considered. The pH buffer aims to maintain the pH constant during the measure of current as a function of potential. The proper pH buffer selection may be performed by working the method of the present invention on different pH buffers. Such pH buffers are commercially available. Preferably, such pH buffers are selected from the group consisting of acetate buffer (pH 3,-5,5), Britton Robinson (pH 2-12), phosphate buffer (pH 7,0-10,0) and ammonia buffer (pH 8,5-10,5).

According to a preferred embodiment, the pH buffer is an acetate buffer (pH 3,-5,5).

The method of the present invention may be implemented, especially step (d), in the presence of at least one electrolyte.

Step (d) has to be implemented in the presence of at least one electrolyte.

Such electrolyte may comprise borate, phosphate, nitrate, chlorine, sodium, hydron, hydroxy ions, or any mixture thereof.

According to one specific embodiment, the electrolyte comprises a nitrate ion, and preferably potassium nitrate (KNO₃).

According to one preferred embodiment, the measure of the current as a function of the potentially is performed after degassing with nitrogen one or more water samples, optionally diluted, and/or one or more concentrated samples.

Advantageously, the method of the present invention may be performed on water containing different types of inhibitors either individually or in mixture.

It was particularly unobvious and surprising for a skilled person that an electroanalytical method involving the measurement of current as a function a potential, such as voltammetry, could be used for dosing or determining the amount of one or more corrosion or scale inhibitors in water..

Corrosion or scale inhibitors are badly characterized because of their complex chemical structure or composition. Therefore it was not obvious that the method of the present invention would work for determining the concentration of such corrosion or scale inhibitors. In particular, such inhibitors comprise surfactant, even several surfactants.

A Corrosion inhibitor designates here specifically a compound capable of reducing the corrosion rate of a material or equipment, typically a metal or metal alloy, due to the action on the material of an oxidant such as oxygen or hydron (H⁺). Typically, corrosion inhibitors may comprise azo compounds, such as alkylimidazolines, and/or thioglycolic acid, one or more diols, such as ethyl-1,2-diol, fatty acids derivatives, alkoxylated alcohols, such as butoxyethanol, ethoxylated amidoamine, ethylene amine, phosphate esters, alcohol such as methanol, or glycols, such as ethylene glycols, and mixtures thereof.

According to one embodiment the method of the present invention aims to determine the concentration of, to control or monitor the concentration of, or to determine the potential of a water sample comprising a corrosion inhibitor comprising alkylimidazoline compounds. Examples of such corrosion inhibitor as described in US 7057050.

Typically such imidazoline corrosion inhibitor presents the formula: 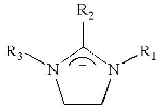 wherein R₁ is an alkyl radical having from 2 to 8 carbon atoms; R₂ is a radical derived from a fatty acid; and R₃ is a radical derived from an acrylic acid

Scale inhibitors are typically compounds inhibiting the deposition of mineral or organic compounds on the surface of equipment in contact with water. It is typically scale inhibitor and/or dispersant. In particular, scale inhibitor means a compound capable of preventing or slowing down the formation of solid mineral salts, for example selected from: calcium, carbonate, calcium sulfate, barium sulfate, stronsium sulfate, zinc sulfide, lead and/or iron and any mixture thereof.

For example scale inhibitors may be selected especially among:
- Polyphosphates - polycarboxylic acids
- Polymers comprising sulfonic acid function,
- Polyethyleneimine (PEI)
- Silicone polymers, especially the polydimethylsiloxanes functionalized with amine groups, and
- Copolymers containing quaternary ammonium, such as copolymers of acrylamide, quaternary ammonium and optionally acrylate and copolymers of acrylamide, diallyldimethylammonium salt and optionally acrylate.

The concentration of corrosion or scale inhibitor is generally of 1 to 1000 ppm, more precisely of 1 to 500 ppm, and more precisely of 10 to 200ppm, in the water sample 10.

In one embodiment concentration of corrosion or scale inhibitor is of 10 to 100 ppm in the water sample 10.

Monitoring of the concentration of corrosion or scale inhibitor may be performed by increasing or decreasing the amount of corrosion or scale inhibitor added in the production equipment.

According to one embodiment, the monitoring is fully automated.

Advantageously, according to one embodiment, it is preferred to dilute the water sample 10 before performing the measure at step (d). Accordingly, in one embodiment, the method of the present invention comprises a step (b) of diluting one or more water samples 10 to prepare diluted samples 20.

Preferably the original water sample 10 is diluted by 10, 20, 30, 40, 50 or more and preferably by 100 or more. Advantageously the dilution improves the measure until it is sufficiently sensitive to corrosion or scale inhibitor amount present in the water sample 10 and that possibly interfering species present in the sample are sufficiently diluted. Advantageously, diluting the original water sample 10 enables limiting the interference of species present in the sample and in particular of oil possibly present (in case of water used for oil production) or salts dissolved in the original water sample 10. Advantageously, by using DPV or SWV, the detection limit is inferior to 0.1 ppm vol. The presence of inhibitors in water, typically of industrial water, and typically water used for gas or oil or gas production, are present at 10 to 100 ppm vol. DPV or SWV has thereby a sufficiently low detection limit to measure the concentration of diluted samples of water containing such low concentration of inhibitors.

Step (d) of measuring the current as a function of the potential of the water sample is performed according to the skilled person knowledge. Typically step (d) comprises collecting at least one current-potential curve (I=f(E); typically a voltammogram) per water sample 10, optionally diluted 20, and/or concentrated 30, and step (e) comprises determining the surface of one or more peaks of the current-potential curve and plotting peak areas as a linear function of different corrosion or scale inhibitor concentrations.

Instead of the peak area, other information obtainable through the current-potential curve (I=f(E); typically a voltammogram) may be used.

The potential is varied between 0 and -1 V, according to one embodiment.

More precisely, the potential is for example varied from -0.05 V to -1.00 V, according to one embodiment.

In one embodiment the potential (E) sweep rate if of 0.1V/s to 3V/s, for example of 0.2V/s.

In one embodiment, the method comprises determining or selecting the highest peak (main peak) of the I=f(E) curve.

According to one embodiment, the selected peak for determining the area thereof is present between -0.1 V and -0.4V.

According to one embodiment, the selected peak for determining the area thereof is present at about -0,2 V.

The peak area is determined according to a method known by the skilled person, and which is typically available through commercially available software programs (ex: Nova 2.0).

According to one embodiment, the concentration of corrosion or scale inhibitor in the original water sample 10 is given by the linear function of peak area as a function of inhibitor concentration.

According to one embodiment, the concentration of corrosion or scale inhibitor in the original water sample 10 is given by the linear function of maximum peak current as a function of inhibitor concentration.

In one embodiment, step (c) comprises adding one or more times a determined amount of one or more corrosion or scale inhibitors 40 to said one or more water samples 10, optionally diluted 20, to prepare one or more concentrated samples 30 with a higher corrosion or scale inhibitor 40 concentration than said water samples 10, optionally diluted 20.

In one embodiment, said method comprises preparing concentrated samples 30 of a water sample 10 and measuring the current as a function of the potential of said sample 10 and of one or more concentrated samples 20.

Advantageously, it is simple to determine this concentration using the standard additions method by adding one or more times a determined amount of the corrosion or precipitation inhibitor(s) (for which the concentration has to be determined), thereby preparing one or more concentrated samples 30 wherein the amount of corrosion or precipitation inhibitor(s) added is known and represents a numerical multiple (1, 2, 3, 4, or 5, for example) of the predetermined amount of inhibitor added. According to a specific embodiment, the concentration of the water sample 10 optionally diluted 20 is determined by the crossing of the linear function of the peak area (or maximum peak current) as a function of added concentration or amount of inhibitor and the ordinate axis (x = 0; i.e. no amount of inhibitor added to the water sample 10 or diluted sample 20).

In one embodiment, if the original sample 10 has been diluted to prepare a diluted sample 20, the dilution factor is taken into account to determine the concentration of inhibitor in the original sample 10.

The present invention also relates to a method for preparing an electrode wherein said method comprises screen printed. Screen printing is performed according to a method known in the art. Such electrodes are made using a carbon ink and ink printing equipment (Olivier Zaouak et al, Electroanalytical Device for Cadmium Speciation in Waters. Part 1: Development and Characterization of a Reliable Screen-Printed Sensor, Electroanalysis 2010, 22, No. 11, 1151 - 1158).

Such carbon screen printed electrodes are cheap, disposable and easily transportable. According to the invention, such electrodes are advantageously recovered by a solid layer of mercury. Mercury can be deposited from a ionic solution containing mercury ion (typically Hg²⁺) by electrodeposition. Such an ionic solution is much less toxic than mercury (metal atom) itself. According to one embodiment, the preparation of the electrode comprises running a current between two electrodes (anode and cathode) while the carbon screen printed electrode is placed in a solution containing Hg²⁺, preferably in an acetate buffer solution, thereby depositing a solid surface of mercury on the carbon electrode surface.

A thin layer of solid mercury is deposited on the surface of the carbon electrode, preferably while the solution is agitated, for example at 400 rpm. In one embodiment, the electric charge deposited on the carbon electrode is of between 1 and 20 mC, for example of 5 mC.

The mercury is deposited on the cathode which is the carbon screen printed electrode.

The layer of mercury deposited has a mass of from 1 to 20 µg. For example, with 5 mC applied deposit has mass of 5 µg.

The electrodes obtained having a solid surface of mercury represent an electrode, especially a working electrode, for use in a method according to the present invention. Accordingly, the present invention relates to an electrode comprising carbon and covered by a solid layer of mercury.

According to a preferred embodiment; the electrode of the invention is prepared by combination of screen printing and electrodeposition.

Accordingly, the method comprises a step of preparing a screen printed carbon electrode having a solid surface layer of mercury, preferably by combination of screen printing a carbon electrode and electrodeposition of said layer of mercury.

By using such an electrode comprising a solid layer of mercury, the technical problem of using liquid mercury as in polarography is overcome and surprisingly the sensitivity and reliability of the method is improved.

The present invention also relates to a method for determining the potential of a water sample 10 comprising a corrosion or scale inhibitor 40, wherein said method comprises preparing at least one electrode comprising a solid surface comprising mercury; putting said electrode (a) in contact with said water samples 10, optionally diluted 20 and/or concentrated sample 30; measuring the current as a function of the potential of said water samples 10, optionally diluted 20, and/or concentrated sample 30, thereby determining the potential of said water sample 10 comprising at least one corrosion or scale inhibitor 40.

The present invention also relates to a method for controlling or monitoring the concentration of one or more corrosion or scale inhibitors 40 in an industrial fluid comprising water and at least one corrosion or scale inhibitor 40, said method comprising implementing a method as defined in the present invention to determine the concentration of said one or more corrosion or scale inhibitors 40 and thereby control or monitor the concentration of said one or more corrosion or scale inhibitors 40 in said industrial fluid.

The present invention also relates to a method for producing gas or petroleum comprising implementing a method for determining a concentration of a corrosion or scale inhibitor 40 in a water sample according to the invention or a method for controlling or monitoring the concentration of one or more corrosion or scale inhibitors 40 in an industrial fluid.

The present invention also relates to a kit for voltammetry analysis, said kit comprising (i) an electrolyte solution, preferably comprising KNO₃, (ii) a pH buffer solution, preferably comprising an acetate buffer solution, optionally (iii) a cleaning solution, for example comprising HNO₃, optionally (iv) one or more standard solutions, preferably comprising a determined corrosion or scale inhibitor at a known or predetermined concentration or amount, and preferably (v) a carbon electrode comprising a solid layer of mercury or a carbon electrode and a solution of Hg. Said kit may be presented in a single packaging or in several packaging.

Other subjects and characteristics, aspects and advantages of the invention will immerge even more clearly on reading the description and the examples that follows.

In that which follows and unless otherwise indicated, the limits of a range of values are included within this range, in particular in the expression "of between" and ranging from ... to ...".

The term "or" means "and/or", thereby "a or b" designates "a", "b", and "a and b", unless contrary expression.

Moreover, the expression "at least one" used in the present invention is equivalent to the expression "one or more".

In the figures:

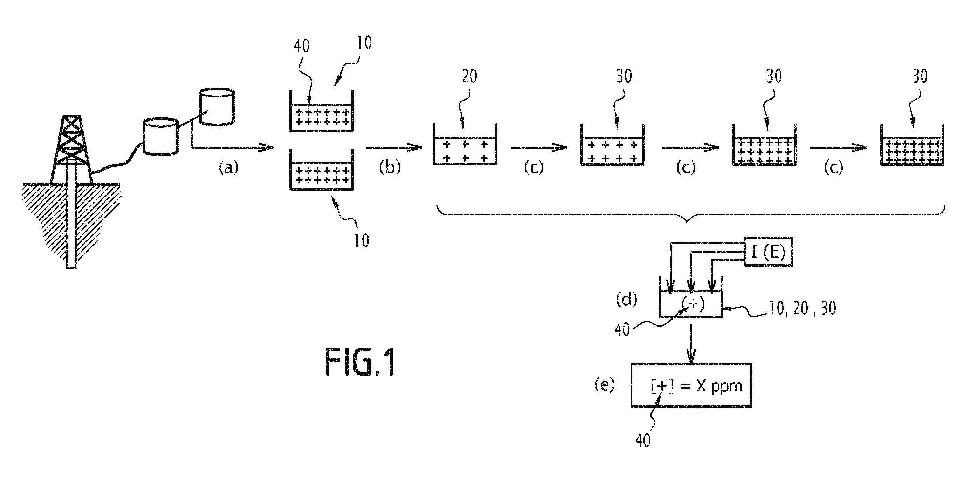

Figure 1 is a schematic representation o the main steps of the method of the invention;

Reference is made to: water samples 10; water diluted samples 20; concentrated samples 30; and corrosion or scale inhibitor 40.
Figure 2 is a graph representing I=f(E) curves of a water sample 10 (of an oil production plant) which has been previously diluted 20 and of 5 different concentrated solutions 30;
Figure 3 is a graph representing the peak area (peak at -0.2V according to fig. 2) as a linear function of the concentration of inhibitor added.

The examples that follow are aimed at illustrating the methods according to this invention, but are in no way a limitation of the scope of the invention, unless contrary stated. In the example the temperature is expressed in °C and is room temperature (22°C) unless contrary expressed.

### EXAMPLES

### Example 1: preparation of a carbon electrode with a solid surface of mercury.

### 1.1 Preparing a mercury ion solution (Hg²⁺) at 5.10⁻³ mol/L

Adding the necessary mass of Hg(CH₃COOH)₂ in a recipient containing ultra-pure water;
Adding the desired volume of CH₃ COOH in the recipient;
Adding the necessary amount of NaOH, H₂O in the recipient;
Agitating and adding the desired amount of ultra-pure water to obtain the desired concentration of Hg²⁺.

### 1.2. Preparing the electrode;

Adding in a metrohm cell the solution containing Hg²⁺ at 5.10⁻³ mol/L (1 mL);
Adding 9 mL of acetate buffer (0.2 mol/L) in the same cell;
Connecting a screen printed carbon electrode to the electrochemical analytical device for use as cathode (for example a screen printed carbon electrode prepared as described in Olivier Zaouak et al, Electroanalytical Device for Cadmium Speciation in Waters. Part 1: Development and Characterization of a Reliable Screen-Printed Sensor, Electroanalysis 2010, 22, No. 11, 1151 - 1158);
Introducing the reference electrode (anode - Ag/AgCl/KCl 3M) in the electrochemical cell containing the Hg²⁺ solution;
Agitating the solution present in the electrochemical cell and adding 20 mL KNO₃ at 10⁻² mol/L in the metrohm cell;
Applying a sufficient current between the anode and cathode during a time sufficient to deliver an electric charge of 5 mC;
After depositing of the desired amount of mercury, rincing the electrode with KNO₃ at 10⁻² mol/L.

A carbon electrode with a thin layer of solid mercury deposited on its surface is obtained.

### Example 2: Measuring the concentration on a sample.

This example illustrates a method according to the invention for determining the concentration of a corrosion or scale inhibitor in an industrial water sample.
pH buffer solution: acetate buffer at 0.2 mol/L;
electrolyte solution: KNO₃ at 10⁻² mol/L solution;
cleaning solution: HNO₃ at 10% solution;
corrosion inhibitor: Norust® 9711T (Arkema);
Working electrode: a screen printed carbon electrode having a thin solid layer of mercury on its surface, such the electrode prepared in example 1;
Counter electrode (auxiliary electrode): screen printed carbon electrode as prepared in example 1, without any mercury layer;
Reference electrode: Ag/AgCl/KCl 3M.

### Preparation of standard solutions:

In a 200 mL recipient, adding about 100 mL of HNO₃ solution at 10⁻² mol/L and 2 mL of the corrosion inhibitor, commercially available;
Making up to the mark with the HNO₃ solution;
Agitating the solution at 400 rpm.

For the tested corrosion inhibitor, the concentration in this concentrated standard solution is of 10000 ppm vol.

The same procedure is reproduced with 2 mL of concentrated standard solution at 10000 ppm vol to prepare a standard solution comprising 40 ppm vol corrosion inhibitor, called "standard solution".

The operating parameters of the voltammetry were as follows:

| | |
|---|---|
| Sweeping of potential | -0.05V to -1.00V |
| Frequency | 25Hz |
| Speed | 0.2V/s |
| Replicates | 10 |
| Volume of supporting electrolyte | 20mL |
| Sample volume | 100µL |

Square wave anodic stripping voltammograms were recorded with an Autolab PGSTAT12 potentiostat (Eco Chemie, The Netherlands) (Squarewave voltammetry (SWV).

The determination of inhibitor concentration is performed as follows:
(i) measuring I=f(E) on diluted water samples 20
   (a) Collecting a water sample 10 from an oil production plant, said water containing a corrosion inhibitor the concentration of which has to be determined.
      Placing said water sample in a metrohm cell.
      Agitating the water sample containing the inhibitor for which the concentration has to be determined at 1000 rpm;
      Degassing with nitrogen the metrohm cell during thirty minutes;
   (b) preparing one or more diluted water samples 20 by diluting 100 times said water sample 10 with deionized water; (d) measuring the current as a function of the potential of on a 100 µL of diluted water sample 20 (diluted solution of water containing the inhibition corrosion for which the concentration has to be determined), with a potential sweep from -0.05 V to -1 V and sweep rate of 0.2V/s, temperature of 22°C (room temperature).
      Collecting the curve I=f(E), where I is the current intensity measured in the metrohm cell, and E the potential applied in the metrohm cell.
(ii) measuring I=f(E) on concentrated water samples 30
   (c) preparing one or more concentrated samples 30 by addition of 100 µL of the standard solution (containing 40 ppm of corrosion inhibitor) to the one or more diluted water sample 20 (first addition - to prepare the concentrated water sample n°1 30), with prior degassing with nitrogen of the metrohm cell during two minutes and
   (d) collecting the current as a function of the potential (I=f(E)) of said concentrated water samples 30;
(iii) Reproducing 4 times (ii) to prepared concentrated water samples 30 with different concentration of corrosion inhibitor.
   (c) preparing one or more concentrated samples 30: adding 100 µL of standard solution (containing 40 ppm of corrosion inhibitor) to the concentrated water sample n°1 to prepare a concentrated water sample n°2 30;
   (c) preparing one or more concentrated samples 30: adding 100 µL of standard solution (containing 40 ppm of corrosion inhibitor) to the concentrated water sample n°2 to prepare a concentrated water sample n°3 30;
   (c) preparing one or more concentrated samples 30: adding 100 µL of standard solution (containing 40 ppm of corrosion inhibitor) to the concentrated water sample n°3 to prepare a concentrated water sample n°4 30;
   (c) preparing one or more concentrated samples 30: adding 100 µL of standard solution (containing 40 ppm of corrosion inhibitor) to the concentrated water sample n°4 to prepare a concentrated water sample n°5 30;

Thereby collecting the curve I=f(E) for concentrated samples n°1, 2, 3, 4 and 5.
(iv) Rinsing the material with the cleaning solution and
(v) Processing the obtained data (I=f(E) of the water sample to be tested and concentrated water samples n°1, 2, 3, 4 and 5).

Processing the data includes determining the peak heights and peaks areas;

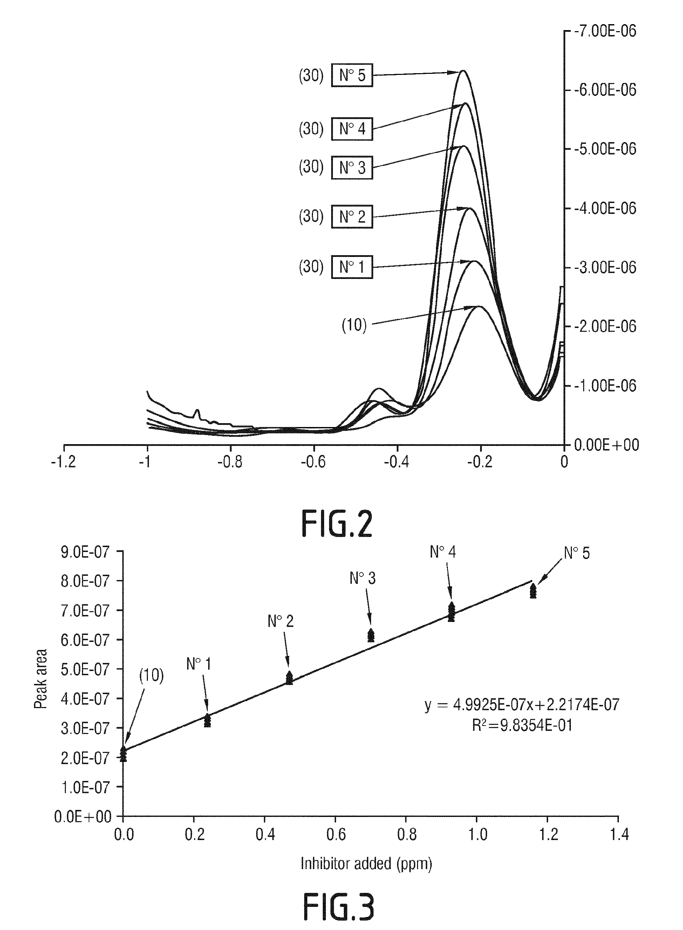

As shown on figure 3, the main peak occurs at -0.2 V and its height increases with addition of the corrosion inhibitor (increase of the concentration of corrosion inhibitor). The selected peak area is calculated by software.

The skilled person would then prepare with a well-known software such as Nova 2.0 a linear function of peak area as a function of corrosion inhibitor concentration (more specifically as a function of added amount of corrosion inhibitor, x=0 representing the concentration of the diluted water sample 20) and determine the concentration of the water sample 10 (which corresponds to x=0 i.e. the point where no corrosion inhibitor has been added to the diluted water sample 20). Dilution factor is taken into account.

According to figure 3, the concentration of corrosion inhibitor tested 10 is of 45 ppm vol. This concentration, as measured by the method of the invention, is similar to the mean dose injected in the equipment and thereby meaningful.

It is thereby possible to determine (easily on site) the concentration of a corrosion or a scale inhibitor in a water sample collected from oil production facilities, monitor the concentration of the corrosion or scale inhibitor in a production equipment.

## Claims

1. A method for determining a concentration of a corrosion or scale inhibitor in a water sample comprising at least one corrosion or scale inhibitor, wherein said method comprises measuring the current as a function of the potential of one or more water samples thereby determining the concentration of said corrosion or scale inhibitor in said water sample.

2. The method of claim 1, wherein said method comprises:
(a) collecting one or more water samples (10) containing water and at least one corrosion or scale inhibitor (40);
(b) optionally diluting one or more water samples (10) to prepare one or more water diluted samples (20), thereby providing one or more water samples (10), optionally diluted (20);
(c) preparing concentrated samples (30) presenting a higher corrosion or scale inhibitor concentration than said water samples (10), optionally diluted (20);
(d) measuring the current as a function of the potential of one or more water samples (10), optionally diluted (20), and of said one or more concentrated samples (30);
(e) thereby determining the concentration of said corrosion or scale inhibitor (30) in said one or more water samples (10).

3. The method of claim 1 or 2, wherein said measure of the current as a function of the potential at step (d) is performed by voltammetry, preferably by polarography, cyclic voltammetry (CV), differential pulse voltammetry (DPV), or squarewave voltammetry (SWV).

4. The method of any one of claims 1 to 3, wherein said measure of the current as a function of the potential at step (d) is performed in the presence of at least one electrode comprising a solid surface comprising mercury.

5. The method of any one of claims 1 to 4, wherein said electrode comprising a solid surface comprising mercury is prepared *in situ,* by mercury electrodeposition on the surface of an electrode, preferably a carbon electrode.

6. The method of any one of claims 1 to 5, wherein step (c) comprises adding one or more times a determined amount of one or more corrosion or scale inhibitors (40) to said one or more water samples (10), optionally diluted (20), to prepare one or more concentrated samples (30) with a higher corrosion or scale inhibitor (40) concentration than said water samples (10), optionally diluted (20).

7. The method of any one of claims 1 to 6, wherein steps (b) and/or (c) comprise adding a buffer solution to said water samples (10), optionally diluted (20).

8. The method of any one of claims 1 to 7, wherein said measure of the current as a function of the potential is performed after degassing with nitrogen one or more water samples (10), optionally diluted (20), and/or one or more concentrated samples (30).

9. The method of any one of claims 1 to 8, wherein said step (d) comprises collecting at least one current-potential curve per water sample (10), optionally diluted (20), and/or concentrated sample (30), and wherein said step (e) comprises determining the area of one or more peaks of the current-potential curve and plotting peak areas as a linear function of different corrosion or scale inhibitor (40) concentrations.

10. The method of any one of claims 1 to 9, wherein the method comprises a step of preparing a screen printed carbon electrode having a solid surface layer of mercury, preferably by combination of screen printing a carbon electrode and electrodeposition of said layer of mercury.

11. A method for determining the potential of a water sample (10) comprising a corrosion or scale inhibitor (40), wherein said method comprises preparing at least one electrode comprising a solid surface comprising mercury; putting said electrode (a) in contact with said water samples (10), optionally diluted (20) and/or concentrated sample (30); measuring the current as a function of the potential of said water samples (10), optionally diluted (20), and/or concentrated sample (30), thereby determining the potential of said water sample (10) comprising at least one corrosion or scale inhibitor (40).

12. A method for controlling or monitoring the concentration of one or more corrosion or scale inhibitors (40) in an industrial fluid comprising water and at least one corrosion or scale inhibitor (40), said method comprising implementing a method as defined in any one of claims 1 to 10 to determine the concentration of said one or more corrosion or scale inhibitors (40) and thereby control or monitor the concentration of said one or more corrosion or scale inhibitors (40) in said industrial fluid.

13. Use of a screen printed carbon electrode comprising a solid surface of mercury (Hg) for measuring the current as a function of the potential of one or more water samples (10), optionally diluted (20) and/or concentrated (30), said sample (10, 20, 30) containing one or more corrosion or scale inhibitor (40).

14. A method for producing gas or petroleum comprising implementing a method as defined in any one of claims 1 to 12.

15. A kit for voltammetry analysis, said kit comprising (i) an electrolyte solution, preferably comprising KNO₃, (ii) a pH buffer solution, preferably comprising an acetate buffer solution, (iii) a carbon electrode comprising a solid layer of mercury or a carbon electrode and a solution of Hg²⁺, for example comprising HNO₃, optionally (iv) one or more standard solutions, preferably comprising a determined corrosion or scale inhibitor at a known or predetermined concentration or amount, and optionally (v) a cleaning solution, preferably comprising HNO₃.
